(19) Europäisches Patentamt
European Patent Office
Office européen des brevets

(11) Numéro de publication : **0 299 891 B1**

# (12) FASCICULE DE BREVET EUROPEEN

(45) Date de publication du fascicule du brevet : **11.12.91 Bulletin 91/50**

(51) Int. Cl.$^5$ : **C07C 37/62**

(21) Numéro de dépôt : **88420243.3**

(22) Date de dépôt : **13.07.88**

## (54) Procédé de chloration sélective de composés phénoliques.

(30) Priorité : **17.07.87 FR 8710418**

(43) Date de publication de la demande : **18.01.89 Bulletin 89/03**

(45) Mention de la délivrance du brevet : **11.12.91 Bulletin 91/50**

(84) Etats contractants désignés : **AT BE CH DE ES FR GB GR IT LI LU NL SE**

(56) Documents cités :
**US-A- 4 160 114**
**PATENT ABSTRACTS OF JAPAN, vol. 11, no. 43 (C-402)[2490], 7 février 1987, page 29 C 402; & JP-A-61 207 351**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière Route de Ternay**
**Communay F-69360 St.-Symphorien D'Ozon (FR)**
Inventeur : **Besson, Bernard**
**15, rue de Moucherotte**
**F-38800 Pont de Claix (FR)**
Inventeur : **Jouve, Isabelle**
**30, rue Léon Fabre**
**F-69100 Villeurbanne (FR)**

(74) Mandataire : **Vignally, Noel et al**
**Rhône-Poulenc Interservices Service Brevets Chimie Centre de Recherches des Carrières**
**B.P. 62**
**F-69192 Saint-Fons Cédex (FR)**

**EP 0 299 891 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la chloration sélective par le chlore gazeux de composés phénoliques, en position ortho par rapport à la fonction hydroxyle.

Dans la préparation des chlorophénols par chloration du phénol, on obtient aux divers stades de la réaction des isomères, ce qui conduit finalement à de nombreux composés, qu'il est ensuite nécessaire de séparer par des opérations coûteuses et délicates. D'autre part, les rapports des différents isomères obtenus ne correspondent pas forcément à ce qui serait économiquement intéressant, compte-tenu des débouchés existants.

Il est donc apparu souhaitable de disposer de procédés de chloration sélective, notamment de chloration sélective en position ortho ou en position para par rapport à la fonction hydroxyle du composé phénolique.

Ainsi la demande de brevet allemand N° 3318791 décrit un procédé sélectif de chloration du phénol en chloro-2 phénol, dans un solvant apolaire perchloré et en présence d'une amine à chaîne ramifiée.

Cependant l'utilisation d'un solvant dans un procédé industriel de chloration peut, dans certains cas, présenter des inconvénients, tels que par exemple la nécessité d'un volume de réacteur plus important et une séparation plus difficile des produits de la réaction.

La présente invention a pour objet un procédé de chloration sélective de composés phénoliques par le chlore gazeux en milieu fondu et en présence d'un cation organique.

Plus précisément il s'agit d'un procédé de chloration sélective en position ortho par rapport à la fonction hydroxyle des composés phénoliques de formule générale (I) :

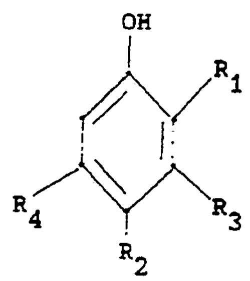

(I)

dans laquelle :

— $R_2$ représente :
- un atome d'halogène ;
- un groupement alkyle ayant 1 à 4 atomes de carbone ;
- un groupement alkoxy ayant 1 à 4 atomes de carbone ;
- un groupement acyloxy ayant 2 à 4 atomes de carbone ;
- un groupement acyle ayant 2 à 4 atomes de carbone ;
- un groupement acide carboxylique ;
- un groupement ester —$COOR_5$, $R_5$ représentant un radical alkyle linéaire ou ramifié ayant de 1 à 4 atomes de carbone ;
- un groupement nitrile ;
- un groupement OH ;
- un groupement —CHO ;
- un groupement —$NO_2$ ;
- un groupement acétamido ;

— $R_1$ représente un atome d'hydrogène ou possède l'une des significations indiquées pour $R_2$.

— $R_3$ et $R_4$, identiques ou différents, représentant un atome d'hyrogène ou un atome ou groupement tel que ceux représentés par $R_2$ ;

en milieu fondu, par le chlore gazeux, caractérisé en ce que l'on opère en présence d'une quantité efficace d'au moins un cation organique.

On peut mettre en oeuvre un cation organique ou un mélange de plusieurs cations organiques.

Par cation organique, on entend dans le présent texte les ions onium dérivant notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène, du carbone ou de S=O seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble

un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581-587 (1968), les termes "dure" et "intermédiaire" ayant respectivement la signification des termes de "hard" et "borderline" utilisés dans cette référence.

Parmi les ions onium pouvant être utilisés dans le présent procédé de chloration, conviennent particulièrement ceux répondant à l'une des formules générales suivantes :

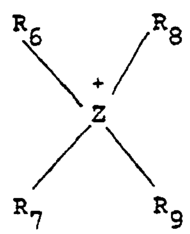

(II)

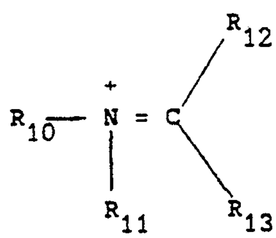

(III)

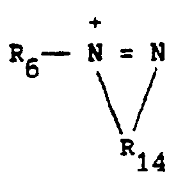

(III bis)

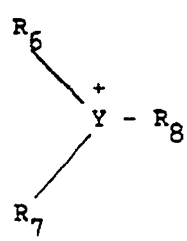

(IV)

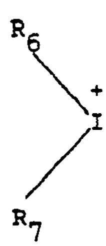

(V)

dans lesquelles :

— Z représente N, P ou As ;

— Y représente S, O, Se, S=O ou C ;

— $R_6$, $R_7$, $R_8$ et $R_9$, identiques ou différents représentent :

• un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

- un radical alcényle, linéaire ou ramifié, ayant 2 à 12 atomes de carbone ;
- un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,
- deux desdits radicaux $R_6$ à $R_9$ pouvant former ensemble un radical alkylène, alcénylène ou alcadiènylène, linéaire ou ramifié ayant de 3 à 6 atomes de carbone ;

— $R_{10}$, $R_{11}$, $R_{12}$, $R_{13}$ sont identiques ou différents et représentent :

- un radical alkyle linéaire ou ramifié contenant de 1 à 4 atomes de carbone ;
- les radicaux $R_{12}$ et $R_{13}$ pouvant former ensemble un radical alkylène contenant de 3 à 6 atomes de carbone ;
- les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

— $R_{14}$ représente un radical divalent formant avec les 2 atomes d'azote un cycle ayant 4 à 6 atomes pouvant comporter un ou plusieurs atomes d'azote, de soufre et/ou d'oxygène ledit cycle pouvant être substitué par un ou plusieurs radicaux tels que $R_6$.

Parmi les bases "dures" ou "intermédiaires" pouvant constituer l'anion desdits sels d'onium, on peut citer les ions : $F^-$, $C10_4^-$,$PF_6^-$, $BF_4^-$, $SnCl_6^-$, $SbCl_6^-$, $B(Ph)_4^-$, $PO_4^{3-}$$HPO_4^{2-}$ $H_2PO_4^-$ $CH_3SO_3^-$ Ph-$SO_3^-$ $HSO_4^-$ $NO_3^-$ $SO_4^{2-}$ $CL^-$, $Br^-$, $I^-$, $OH^-$, Ph représentant un radical phényle, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$ $HPO_4^{2-}$$H_2PO_4^-$ $CH_3SO_3^-$ Ph-$SO_3^-$$NO_3^-$ $SO_4^{2-}$ $PF_6^-$, $Cl^-$, $Br^-$, $I^-$,
Ph ayant la signification précédente. On recourra avantageusement aux anions $Cl^-$ et $Br^-$ et plus particulièrement à l'anion $Cl^-$.

A titre d'exemples de cations organiques répondant à la formule (II) on peut citer les cations :
tétraméthylammonium,
— triéthylméthylammonium,
— tributylméthylammonium,
— triméthylpropylammonium,
— tétraéthylammonium,
— tétrabutylammonium,
— dodécyltriméthylammonium,
— méthyltrioctylammonium,
— heptyltributylammonium,
— tétrapropylammonium,
— tétrapentylammonium,
— tétrahexylammonium,
— tétraheptylammonium,
— tétraoctylammonium,
— tétradécylammonium,
— butyltripropylammonium,
— méthyltributylammonium,
— pentyltributylammonium,
— méthyldiétylpropylammonium,
— éthyldiméthylpropylammonium,
— tétradodécylammonium,
— tétraoctadécylammonium,
— hexadécyltriméthylammonium,
— benzyltriméthylammonium,
— benzyldiméthylpropylammonium,
— benzyldiméthyloctylammonium,
— benzyltributylammonium,
— benzyltriéthylammonium,
— phényltriméthylammonium,
— benzyldiméthyltétradécylammonium,
— benzyldiméthylhexadécylammonium,
— diméthyldiphénylammonium,

— méthyltriphénylammonium,
— butène-2-yltriéthylammonium,
— N,N-diméthyl-tétraméthylèneammonium,
— N,N-diéthyl-tétraméthylènammonium,
— tétraméthylphosphonium,
— tétrabutylphosphonium,
— éthyltriméthylphosphonium,
— triméthylpentylphosphonium,
— octyltriméthylphosphonium,
— dodécyltriméthylphosphonium,
— triméthylphénylphosphonium,
— diéthyldiméthylphosphonium,
— dicyclohexeyldiméthylphosphonium,
— diméthyldiphénylphosphonium,
— cyclohexyltriméthylphosphonium,
— triéthylméthylphosphonium,
— méthyltri(isopropyl)phosphonium, — méthyltri(n-propyl)phosphonium,
— méthyltri(n-butyl)phosphonium,
— méthyltri(méthyl-2 propyl)phosphonium,
— méthyltricyclohexylphosphonium,
— méthyltriphénylphosphonium,
— méthyltribenzylphosphonium,
— méthyltri(méthyl-4 phényl) phosphonium,
— méthyltrixylylphosphonium,
— diéthylméthylphénylphosphonium,
— dibenzylméthylphénylphosphonium,
— éthyltriphénylphosphonium,
— tétraéthylphosphonium,
— éthyltri(n-propyl) phosphonium,
— triéthylpentylphosphonium,
— hexadécyltributylphosphonium,
— éthyltriphénylphosphonium,
— n-butyltri(n-propyl)phosphonium,
— butyltriphénylphosphonium,
— benzyltriphénylphosphonium,
— (β-phényléthyl)diméthylphénylphosphonium,
— tétraphénylphosphonium,
— triphényl(méthyl-4 phényl)phosphonium,
— tétrakis(hydroxyméthyl)phosphonium,
— tétraphénylarsonium.

Parmi les cations répondant aux formules (III) et (III bis) on peut citer les cations :

— N-méthylpyridinium,
— N-éthylpyridinium,
— N-hexadécylpyridinium,
— N-méthylpicolinium,
— triphényl-1,2,4 triazolium.

A titre d'exemples de cations organiques répondant à la formule (IV), on peut citer les cations :

— triméthylsulfonium,
— triéthylsulfonium,
— triphénylsulfonium,
— triméthylsulfoxonium,
— triphénylcarbénium,
— triéthyloxonium.

A titre d'exemples de cations organiques répondant à la formule (V), on peut citer les cations :

— diphényliodonium,
— diméthoxy-4,4′ diphényliodonium (ou les composés décrits dans JACS 1959, 81, 342),
— diphényliodonium 2-carboxylate

Parmi les cations organiques qui peuvent être utilisés dans le cadre du présent procédé, on préfèrera le

plus souvent les ions ammonium quaternaire, les ions phosphonium quaternaire, les ions sulfonium et les ions iodonium.

La quantité de cation organique utilisée dans le présent procédé peut varier dans de très larges limites.

Habituellement elle représente, en poids de sel d'onium par rapport au poids de composé phénolique (I) de 0,005% à 25%. De préférence on mettra en oeuvre des quantités de cation organique, représentant en poids de sel d'onium par rapport au poids de composé phénolique de formule (I), de 0,01% à 5%.

Le sel d'onium peut être introduit à l'état solide ou sous forme d'une solution dans l'un de ses solvants, le plus souvent l'eau.

Les composés phénoliques de formule (I), pour lesquels le présent procédé offre le plus d'intérêt, sont ceux dans la formule desquels :

— $R_2$ représente :
  - un atome de chlore, un atome de fluor ou un atome de brome ;
  - un groupement méthyle, éthyle, propyle, isopropyle ou tertiobutyle ;
  - un groupement méthoxy ou éthoxy ;
  - un groupement aldéhyde ;
  - un groupement OH ;
  - un groupement CN ;
  - un groupement $NO_2$ ;
  - un groupement acétoxy ;
  - un groupement acétamido ;
  - un groupement ester —$COOR_5$, $R_5$ représentant un groupement méthyle ou éthyle ;
  - un groupement acyle ayant 2 à 4 atomes de carbone ;

— $R_1$ représente un atome d'hydrogène ou a les significations indiquées pour $R_2$

— $R_3$ et $R_4$ représentent :
  - l'un un atome d'hydrogène ;
  - l'autre un atome d'hydrogène ou un groupement tel que ceux représentés par $R_2$.

A titre d'exemples de composés phénoliques de formule (I), on peut citer notamment : le chloro-4 phénol, le dichloro-2,4 phénol, le dichloro-3,4 phénol, le chloro-2 fluoro-4 phénol, le chloro-4 fluoro-2 phénol, le bromo-2 chloro-4 phénol, le bromo-4 chloro-2 phénol, le chloro-2 méthoxy-4 phénol, le chloro-4 méthoxy-2 phénol, le chloro-4 méthyl-2 phénol, le méthoxy-4 phénol, l'éthoxy-4 phénol, l'hydroxy-4 acétophénone, l'hydroxy-4 benzonitrile, le chloro-3 hydroxy-4 benzaldéhyde, le chloro-5 hydroxy-2 benzaldéhyde, le chloro-2 méthyl-4 phénol, le chloro-2 tertiobutyl-4 phénol, le chloro-2 nitro-4 phénol, le chloro-4 nitro-2 phénol, le nitro-4 phénol, le dinitro-2,4 phénol, l'acétamido-4 chloro-2 phénol, l'acétamido-2 chloro-4 phénol, le tertiobutyl-4 phénol, le méthyl-4 phénol, l'éthyl-4 phénol, l'isopropyl-4 phénol.

Le présent procédé possède plusieurs avantages.

Un de ces avantages consiste dans sa sélectivité intramoléculaire : lorsqu'il est appliqué à un composé phénolique de formule (I) dans laquelle au moins une des positions en méta de la fonction hydroxyle est libre, pratiquement seules les positions en ortho de l'OH sont chlorées, alors qu'en l'absence de cation organique il se forme toujours une quantité non négligeable du chloro-3 phénol ou du chloro-5 phénol correspondant, qu'il est ensuite très difficile de séparer. En outre ces composés peuvent être très gênants pour certaines applications.

Un autre avantage très intéressant concerne la sélectivité intermoléculaire du procédé : si le procédé est mis en oeuvre avec un mélange comportant un composé phénolique de formule (I) et un ou plusieurs autres composés phénoliques (tels que par exemple des isomères de position dudit composé phénolique de formule (I)) ne répondant pas à la formule (I), c'est en grande majorité le composé phénolique de formule (I) qui est chloré, tandis que le ou les autres composés phénoliques ne sont que peu transformés.

Ainsi par exemple le procédé de l'invention peut avantageusement être appliqué à un mélange de dichloro-2,4 phénol et de dichloro-2,6 phénol ; on obtient du trichloro-2,4,6 phénol à partir du dichloro-2,4 phénol tandis que le dichloro-2,6 phénol, qui n'est que peu transformé, peut être relativement facilement séparé du mélange réactionnel final.

On peut également mettre en oeuvre un mélange brut industriel contenant du dichloro-2,4 phénol, du dichloro-2,6 phénol et du trichloro-2,4,6 phénol et obtenir ainsi comme précédemment du trichloro-2,4,6 phénol et du dichloro-2,6 phénol, qui peuvent être séparés par distillation fractionnée.

Enfin un autre intérêt du présent procédé tient à ce que la réaction du chlore gazeux et du composé phénolique de formule (I) est pratiquement complète, alors qu'en l'absence de cation organique une partie importante du chlore ne réagit pas. Les problèmes de recyclage de l'excès de chlore ou de traitement des effluents gazeux deviennent ainsi moins difficiles à résoudre.

La quantité de chlore utilisée dans le procédé de l'invention est par conséquent essentiellement fonction

du taux de transformation souhaité du composé phénolique (I).

Le chlore peut être utilisé seul ou être dilué par un gaz inerte, tel que l'azote par exemple. La présence d'un gaz inerte permet, si nécessaire, d'augmenter le débit gazeux sans augmenter la quantité de chlore introduite en un temps donné.

La température à laquelle est mis en oeuvre le procédé est généralement inférieure ou égale à 150°C. D'autre part la limite inférieure de la zone de température est conditionnée par le point de fusion du composé phénolique (I) utilisé ou du mélange de composé phénoliques.

Généralement cette température est comprise entre le point de fusion du mélange réactionnel et 120°C, mais sans que ces chiffres aient une valeur critique.

Les exemples qui suivent ont pour but d'illustrer le procédé de l'invention.

## EXEMPLES 1 à 9

Dans un réacteur en verre de 250 $cm^3$, équipé d'un agitateur à palettes, d'une tubulure permettant l'arrivée du chlore gazeux, d'un thermomètre et surmonté d'un réfrigérant relié à une colonne de destruction du chlore, on introduit les charges suivantes :

— dichloro-2,4 phénol : 21,19 g (130 millimoles)
— dichloro-2,6 phénol : 21,19 g (130 millimoles)
— sel d'onium : nature et quantité indiquées dans le tableau ci-après.

On porte la température à 70°C sous agitation de façon à ce que le mélange soit à l'état liquide et on commence alors à introduire le chlore gazeux avec un débit de 5 litres/heures.

La durée de la chloration effectuée à 70°C est de 33 minutes, ce qui correspond à 2,76 litres de chlore introduit (123,5 millimoles). L'essai est donc réalisé avec un défaut de chlore de 5% par rapport au dichloro-2,4 phénol.

En fin de réaction, on purge l'ensemble de l'installation par un courant d'azote. On obtient une masse réactionnelle que l'on analyse par chromatographie en phase vapeur en présence d'un étalon interne.

Le tableau ci-après rassemble les résultats obtenus.

TABLEAU

| ESSAIS | SEL D'ONIUM UTILISE COMME CATALYSEUR | QUANTITE DE SEL D'ONIUM/ DCP- 2,4 % P/P | CHLORE (en moles) | | TT % du | | RT % en TCP-2,4,6 | RAPPORT TT DCP-2,4 / TT DCP-2,6 |
|---|---|---|---|---|---|---|---|---|
| | | | engagé | fixé | DCP-2.4 | DCP-2.6 | | |
| Témoin A | Néant | 0 | 0,123 | 0,107 | 47,2 | 38,6 | 99,9 | 1,2 |
| Exemple 1 | Chlorure de tétrabutyl-ammonium | 0,5 | 0,123 | 0,112 | 76,5 | 14,5 | 100 | 5.3 |
| Exemple 2 | Chlorure de benzyl-triméthyl-hexadécyl-ammonium | 0,75 | 0,123 | 0,121 | 85,4 | 12,0 | 100 | 7,1 |
| Exemple 3 | Iodure de tétraphényl-phosphonium | 0,5 | 0,123 | 0,119 | 54,9 | 23,9 | 99.5 | 2,3 |
| Exemple 4 | Chlorure de méthyl-triphényl-phosphonium | 0,5 | 0,123 | 0,115 | 76,2 | 19,1 | 98,2 | 4,0 |

% P/P = pourcentage en poids par poids
TT = taux de transformation
DCP-2,4 = dichloro-2,4 phénol
DCP-2,6 = dichloro-2,6 phénol
RT = rendement par rapport au dichlorophénol transformé
TCP-2,4,6 = trichloro-2,4,6 phénol

TABLEAU (suite)

| ESSAIS | SEL D'ONIUM UTILISE COMME CATALYSEUR | QUANTITE DE SEL D'ONIUM/ DCP- 2,4 % P/P | CHLORE (en moles) | | TT % du | | RT % en TCP-2,4,6 | RAPPORT TT DCP-2,4 / TT DCP-2,6 |
|---|---|---|---|---|---|---|---|---|
| | | | engagé | fixé | DCP-2,4 | DCP-2,6 | | |
| Exemple 5 | Bromure de tétrabutyl-phosphonium | 0,5 | 0,123 | 0,119 | 80,7 | 13,4 | 98,4 | 6,0 |
| Exemple 6 | Chlorure de tétraphényl-arsonium | 0,5 | 0,123 | 0,119 | 71,1 | 26,1 | 94,5 | 2,7 |
| Exemple 7 | Chlorure de diphényl-iodonium | 0,5 | 0,123 | 0,120 | 87,6 | 7,5 | 97,1 | 11,7 |
| Exemple 8 | Chlorure de triphényl-sulfonium (solution aqueuse à 50 % P/P | 0,5 | 0,123 | 0,119 | 76,2 | 19,3 | 98,9 | 3,9 |

% P/P = pourcentage en poids par poids
TT = taux de transformation
DCP-2,4 = dichloro-2,4 phénol
DCP-2,6 = dichloro-2,6 phénol
RT = rendement par rapport au dichlorophénol transformé
TCP-2,4,6 = trichloro-2,4,6 phénol

EXEMPLE 9

Dans l'appareillage décrit pour les exemples 1 à 8, on charge :
— dichloro-2,4 phénol : 32,6 g (200 millimoles)
— chlorure de tétrabutylammonium : 0,3 g (environ 1% en poids par rapport au dichlorophénol).
le mélange est chauffé à 70°C sous agitation de manière à ce qu'il soit liquide, puis on introduit du chlore gazeux avec un débit de 5 litres/heures.

La durée de la chloration effectuée à 70°C est de 54 min, ce qui correspond à l'introduction de 200 millimoles de chlore.

On traite l'essai comme indiqué pour les exemples 1 à 8. Un essai témoin B est effectué dans les mêmes conditions sans chlorure de tétrabutylammonium. Afin d'avoir un taux de transformation (TT) du dichloro-2,4 phénol suffisant, il est nécessaire d'introduire un excès de chlore d'environ 60 %, par rapport à la quantité stoechiométrique.

On obtient les résultats suivants :

Exemple 9 :

TT du dichloro-2,4 phénol : 100%
RT en trichloro-2,4,6 phénol : 99,3%

Témoin B

TT du dichloro-2,6 phénol : 85,0%
RT en trichloro-2,4,6 phénol : 97,0%

## Revendications

1. Procédé de chloration sélective en position ortho par rapport à la fonction hydroxyle des composés phénoliques de formule générale (I) :

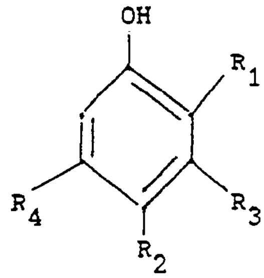

(I)

dans laquelle :

— $R_2$ représente :

- un atome d'halogène ;
- un groupement alkyle ayant 1 à 4 atomes de carbone ;
- un groupement alkoxy ayant 1 à 4 atomes de carbone ;
- un groupement acyloxy ayant 2 à 4 atomes de carbone ;
- un groupement acyle ayant 2 à 4 atomes de carbone ;
- un groupement acide carboxylique ;
- un groupement ester —$COOR_5$, $R_5$ représentant un radical alkyle linéaire ou ramifié ayant de 1 à 4 atomes de carbone ;
- un groupement nitrile ;
- un groupement OH ;
- un groupement —CHO ;
- un groupement —$NO_2$ ;
- un groupement acétamido ;

— $R_1$ représente un atome d'hydrogène ou possède l'une des significations indiquées pour $R_2$ ;

— $R_3$ et $R_4$, identiques ou différents, représentent un atome d'hydrogène ou un atome ou groupement tel que ceux représentés par $R_2$ ;

en milieu fondu, par le chlore gazeux, caractérisé en ce que l'on opère en présence d'une quantité efficace d'au moins un cation organique.

2. Procédé selon la revendication 1, caractérisé en ce que l'on opère en présence d'au moins un cation organique choisi parmi les ions onium répondant à l'une des formules générales suivantes :

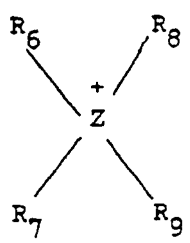

(II)

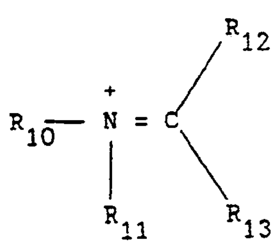

(III)

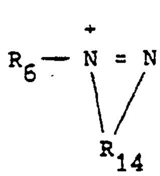

(III bis)

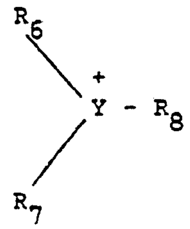

(IV)

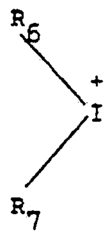

(V)

dans lesquelles :

— Z représente N, P ou As ;

— Y représente S, O, Se, S=O ou C ;

— $R_6$, $R_7$, $R_8$ et $R_9$, identiques ou différents représentent :

- un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
- un radical alcényle, linéaire ou ramifié, ayant 2 à 12 atomes de carbone ;
- un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,
- deux desdits radicaux $R_6$ à $R_9$ pouvant former ensemble un radical alkylène, alcénylène ou alcadiènylène, linéaire ou ramifié ayant de 3 à 6 atomes de carbone ;

— $R_{10}$, $R_{11}$, $R_{12}$, $R_{13}$ sont identiques ou différents et représentent :

- un radical alkyle linéaire ou ramifié contenant de 1 à 4 atomes de carbone ;
- les radicaux $R_{12}$ et $R_{13}$ pouvant former ensemble un radical alkylène contenant de 3 à 6 atomes de carbone ;
- les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

— $R_{14}$ représente un radical divalent formant avec les 2 atomes d'azote un cycle ayant 4 à 6 atomes pouvant comporter un ou plusieurs atomes d'azote, de soufre et/ou d'oxygène ledit cycle pouvant être substitué par un ou plusieurs radicaux tels que $R_6$.

3. Procédé selon l'une des revendications 1 à 2, caractérisé en ce que l'anion lié aux cations organiques est choisi parmi les ions : $F^-$, $ClO_4^-$, $PF_6^-$, $BF_4^-$, $SnCl_6^-$, $SbCl_6^-$,$B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle.

4. Procédé selon l'une des revendications 1 ou 2 caractérisé en ce que l'anion lié aux cations organiques est choisi de préférence parmi les ions : $PO_4^{3-}$ ; $HPO_4^{2-}$ ; $H_2PO_4^-$ ; $CH_3SO_3^-$ · $Ph\text{-}SO_3^-$ · $NO_3^-$ · $SO_4^{2-}$ ; $PF_6^-$ ; $Cl^-$ ; $Br^-$ ; $I^-$ ;
Ph représentant un radical phényle.

5. Procédé selon l'une des revendications 1 à 4, caractérisé en ce que le cation organique est de préférence un ammonium quaternaire, un phosphonium quaternaire, un sulfonium ou un iodonium.

6. Procédé selon l'une des revendications 1 à 5 caractérisé en ce que le cation organique est présent en quantité telle que le poids de sel d'onium par rapport au poids de composé phénolique de formule (I) varie de 0,005% à 25%.

7. Procédé selon l'une des revendications 1 à 5, caractérisé en ce que le cation organique est présent en quantité telle que le poids de sel d'onium par rapport au poids du composé phénolique de formule (I) varie de 0,01% à 5%.

8. Procédé selon l'une des revendications 1 à 7, caractérisé en ce qu'il est appliqué aux composés phénoliques de formule générale (I) dans laquelle :

— $R_2$ représente :
- un atome de chlore, un atome de fluor ou un atome de brome ;
- un groupement méthyle, éthyle, propyle, isopropyle ou tertiobutyle ;
- un groupement méthoxy ou éthoxy ;
- un groupement aldéhyde ;
- un groupement OH ;
- un groupement CN ;
- un groupement $NO_2$ ;
- un groupement acétoxy ;
- un groupement acétamido ;
- un groupement ester —$COOR_5$, $R_5$ représentant un groupement méthyle ou éthyle ;
- un groupement acyle ayant 2 à 4 atomes de carbone ;

— $R_1$ représente un atome d'hydrogène ou a les significations indiquées pour $R_2$ ;

— $R_3$ et $R_4$ représentent :
- l'un un atome d'hydrogène ;
- l'autre un atome d'hydrogène ou un groupement tel que ceux représentés par $R_2$.

9. Procédé selon l'une des revendications 1 à 8, caractérisé en ce qu'il est appliqué aux composés phénoliques choisis parmi le chloro-4 phénol, le dichloro-2,4 phénol, le dichloro-3,4 phénol, le chloro-2 fluoro-4 phénol, le chloro-4 fluoro-2 phénol, le bromo-2 chloro-4 phénol, le bromo-4 chloro-2 phénol, le chloro-2 méthoxy-4 phénol, le chloro-4 méthoxy-2 phénol, le chloro-4 méthyl-2 phénol, le méthoxy-4 phénol, l'éthoxy-4 phénol, l'hydroxy-4 acétophénone, l'hydroxy-4 benzonitrile, le chloro-3 hydroxy-4 benzaldéhyde, le chloro-5 hydroxy-2 benzaldéhyde, le chloro-2 méthyl-4 phénol, le chloro-2 tertiobutyl-4 phénol, le chloro-2 nitro-4 phénol, le chloro-4 nitro-2 phénol, le nitro-4 phénol, le dinitro-2,4 phénol, l'acétamido-2 chloro-4 phénol, l'acétamido-4 chloro-2 phénol, le tertiobutyl-4 phénol, le méthyl-4 phénol, l'éthyl-4 phénol, l'isopropyl-4 phénol.

10. Procédé selon l'une des revendications 1 à 9, caractérisé en ce qu'il est appliqué à un mélange de dichloro-2,4 phénol et de dichloro-2,6 phénol.

11. Procédé selon l'une des revendications 1 à 10, caractérisé en ce qu'il est appliqué à un mélange industriel contenant du dichloro-2,4 phénol, du dichloro-2,6 phénol et du trichloro-2,4,6 phénol.

12. Procédé selon l'une des revendications 1 à 11, caractérisé en ce qu'il est mis en oeuvre à une température ou égale à 150°C et de préférence entre le point de fusion du mélange réactionnel et 120°C.

**Patentansprüche**

1. Verfahren der selektiven Chlorierung von Phenolverbindungen der allgemeinen Formel (I)

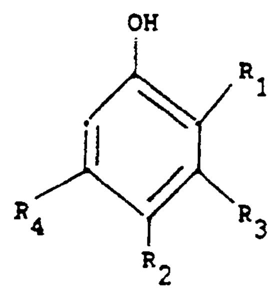

(I)

in der

— $R_2$
- ein Halogenatom,
- eine Alkylgruppe mit 1 bis 4 Kohlenstoffatomen,
- eine Alkoxygruppe mit 1 bis 4 Kohlenstoffatomen,
- eine Acyloxygruppe mit 2 bis 4 Kohlenstoffatomen,
- eine Acylgruppe mit 2 bis 4 Kohlenstoffatomen,
- eine Carbonsäuregruppe,
- eine Estergruppe —$COOR_5$, wobei $R_5$ einen linearen oder verzweigten Alkylrest mit 1 bis 4 Kohlenstoffatomen bedeutet,
- eine Nitrilgruppe,

- eine OH-Gruppe,
- eine CHO-Gruppe,
- eine $NO_2$ -Gruppe,
- eine Acetamidogruppe bedeutet,

— $R_1$ für ein Wasserstoffatom steht oder eine der für $R_2$ angegebenen Bedeutungen aufweist,

— $R_3$ und $R_4$ gleich oder verschieden, für ein Wasserstoffatom oder ein Atom oder eine Gruppe, wie für $R_2$ angegeben, stehen, in Orthostellung, bezogen auf die Hydroxylgruppe, in geschmolzenem Medium, mittels Chlorgas, dadurch gekennzeichnet, daß man in Gegenwart einer wirksamen Menge mindestens eines organischen Kations arbeitet.

2. Verfahren nach Anspruch 1, dadurch gekennzeichnet, daß man in Gegenwart mindestens eines organischen Kations arbeitet, das aus den Oniumionen ausgewählt ist, die einer der folgenden allgemeinen Formeln entsprechen :

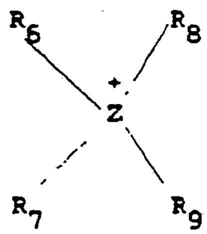
(II)

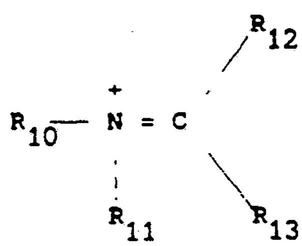
(III)

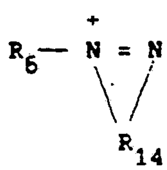
(III bis)

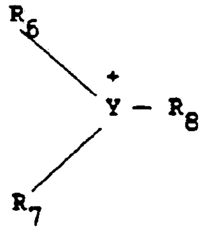
(IV)

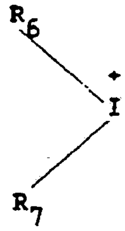
(V)

in denen

— Z für N, P oder As steht,

— Y für S, O, Se, S=O oder C steht,

— $R_6$, $R_7$, $R_8$ und $R_9$ gleich oder verschieden sind und bedeuten :

• einen linearen oder verzweigten Alkylrest mit 1 bis 16 Kohlenstoffatomen, gegebenenfalls substituiert durch eine/ein oder mehrere der folgenden Gruppen oder Atome Phenyl, Hydroxyl, Halogen, Nitro, Alkoxy oder Alkoxycarbonyl, wobei die Alkoxygruppen 1 bis 4 Kohlenstoffatome enthalten,

• einen linearen oder verzweigten Alkenylrest mit 2 bis 12 Kohlenstoffatomen,

• einen Arylrest mit 6 bis 10 Kohlenstoffatomen, gegebenenfalls substituiert durch eine/ein oder mehrere der folgenden Gruppen oder Atome Alkyl mit 1 bis 4 Kohlenstoffatomen, Alkoxy, Alkoxycarbonyl, wobei der Alkoxyrest 1 bis 4 Kohlenstoffatome enthält, oder Halogen,

• zwei der Reste $R_6$ bis $R_9$ zusammen einen linearen oder verzweigten Alkylen-, Alkenylen- oder Alkadienylenrest mit 3 bis 6 Kohlenstoffatomen bilden können,

— $R_{10}$, $R_{11}$, $R_{12}$, $R_{13}$ gleich oder verschieden sind und bedeuten :

• einen linearen oder verzweigten Alkylrest mit 1 bis 4 Kohlenstoffatomen,

• die Reste $R_{12}$ und $R_{13}$ zusammen einen Alkylenrest mit 3 bis 6 Kohlenstoffatomen bilden können,

• die Reste $R_{11}$ und $R_{12}$ oder $R_{11}$ und $R_{13}$ zusammen einen Alkylen-, Alkenylen- oder Alcadienylenrest mit 4 Kohlenstoffatomen bilden können, der mit dem Stickstoffatom einen stickstoffhaltigen Heterozyklus bildet,

— $R_{14}$ einen zweiwertigen Rest, der mit den 2 Stickstoffatomen einen Zyklus bildet, der 4 bis 6 Atome aufweist, die ein oder mehrere Stickstoff-, Schwefel- und/oder Sauerstoffatome einschließen können, wobei dieser Zyklus durch einen oder mehrere Reste, wie $R_6$, substituiert sein kann.

3. Verfahren nach einem der Ansprüche 1 bis 2, dadurch gekennzeichnet, daß das an das organische Kation gebundene Anion ausgewählt wird aus : $F^-$, $C10_4^-$, $PF_6^-$, $BF_4^-$, $SnCl_6^-$, $SbCl_6^-$, $B(Ph)_4^-$, $PO_4^{3-}$, $HPO_4^{2-}$, $CH_3SO_3^-$, Ph-$SO_3^-$, $HSO_4^-$, $NO_3^-$, $SO_4^{2-}$, $Cl^-$, $Br^-$, $I^-$, $OH^-$, wobei Ph einen Phenylrest bedeutet.

4. Verfahren nach einem der Ansprüche 1 oder 2, dadurch gekennzeichnet, daß das an das organische Kation gebundene Anion aus folgenden Ionen ausgewählt ist: $PF_6^-$, $PO_4^{3-}$, $HPO_4^{2-}$, $H_2PO_4^-$, $CH_3SO_3^-$, Ph-$SO_3^-$, $NO_3^-$, $SO_4^{2-}$, $Cl^-$, $Br^-$, $I^-$, wobei Ph einen Phenylrest bedeutet.

5. Verfahren nach einem der Ansprüche 1 bis 4, dadurch gekennzeichnet, daß das organische Kation vorzugsweise ein quaternäres Ammonium, ein quaternäres Phosphonium, ein Sulfonium oder ein Iodonium ist.

6.Verfahren nach einem der Ansprüche 1 bis 5, dadurch gekennzeichnet, daß das organische Kation in solcher Menge vorhanden ist, daß das Gewicht des Oniumsalzes, bezogen auf die Phenolverbindung der Formel (I), 0,005 bis 25 Gew.-% ausmacht.

7. Verfahren nach Anspruch 1 bis 5, dadurch gekennzeichnet, daß das organische Kation in solcher Menge vorhanden ist, daß das Gewicht des Oniumsalzes, bezogen auf die Phenolverbindung der Formel (I), 0,01 bis 5 Gew.-% ausmacht.

8. Verfahren nach einem der Ansprüche 1 bis 7, dadurch gekennzeichnet, daß es auf Phenolverbindungen der allgemeinen Formel (I) angewandt wird, in der

— $R_2$

• ein Chlor-, Fluor- oder Bromatom,

• eine Methyl-, Ethyl-, Propyl-, Isopropyl- oder tert.Butylgruppe,

• eine Methoxy- oder Ethoxygruppe,

• eine Aldehydgruppe,

• eine OH-Gruppe,

• eine CN-Gruppe,

• eine $NO_2$-Gruppe,

• eine Acetoxygruppe,

• eine Acetamidogruppe,

• eine Estergruppe —$COOR_5$, wobei $R_5$ für eine Methyl- oder Ethylgruppe steht, oder

• eine Acylgruppe mit 2 bis 4 Kohlenstoffatomen bedeutet,

— $R_1$ für ein Wasserstoffatom steht oder eine der für $R_2$ angegebenen Bedeutungen hat,

— $R_3$ und $R_4$ bedeuten :

• eines ein Wasserstoffatom,

• das andere ein Wasserstoffatom oder eine Gruppe, wie für $R_2$ angegeben.

9. Verfahren nach einem der Ansprüche 1 bis 8, dadurch gekennzeichnet, daß es auf Phenolverbindungen angewandt wird, die ausgewählt werden unter 4-Chlorphenol, 2,4-Dichlorphenol, 3,4-Dichlorphenol, 2-Chlor-4-fluorphenol, 4-Chlor-2-fluorphenol, 2-Brom-4-chlorphenol, 4-Brom-2-chlorphenol, 2-Chlor-4-methoxyphenol, 4-Chlor-2-methoxyphenol, 4-Chlor-2-methylphenol, 4-Methoxyphenol, 4-Ethoxyphenol, 4-Hydroxyacetophenon, 4-Hydroxybenzonitril, 3-Chlor-4-hydroxybenzaldehyd, 5-Chlor-2-hydroxybenzaldehyd, 2-Chlor-4-methylphenol, 2-Chlor-4-tert.butylphenol, 2-Chlor-4-nitrophenol, 4-Chlor-2-nitrophenol, 4-Nitrophenol, 2,4-Dinitrophenol, 2-Acetamido-4-chlorphenol, 4-Acetamido-2-chlorphenol, 4-tert.Butylphenol, 4-Methylphenol, 4-Ethylphenol, 4-Isopropylphenol.

10. Verfahren nach einem der Ansprüche 1 bis 9, dadurch gekennzeichnet, daß es auf ein Gemisch aus 2,4-Dichlorphenol und 2,6-Dichlorphenol angewandt wird.

11. Verfahren nach einem der Ansprüche 1 bis 10, dadurch gekennzeichnet, daß es auf ein technisches Gemisch angewandt wird, das 2,4-Dichlorphenol, 2,6-Dichlorphenol und 2,4,6-Trichlorphenol enthält.

12. Verfahren nach einem der Ansprüche 1 bis 11, dadurch gekennzeichnet, daß es bei einer Temperatur unter oder gleich 150°C, vorzugsweise zwischen dem Schmelzpunkt des Reaktionsgemisches und 120°C durchgeführt wird.

## Claims

1. Process for the selective chlorination, in the ortho position relative to the hydroxyl group, of the phenolic compounds of general formula (I) :

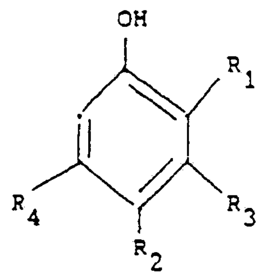

(I)

in which :

— $R_2$ denotes :

a halogen atom ;
an alkyl group having 1 to 4 carbon atoms ;
an alkoxy group having 1 to 4 carbon atoms ;
an acyloxy group having 2 to 4 carbon atoms ;
an acyl group having 2 to 4 carbon atoms ;
a carboxylic acid group ;
an ester group $-COOR_5$, $R_5$ denoting a straight-chain or branched alkyl radical having 1 to 4 carbon atoms ;
a nitrile group ;
an OH group ;
a $-CHO$ group ;
an $-NO_2$ group ;
an acetamido group ;

— $R_1$ denotes a hydrogen atom or has one of the meanings indicated for $R_2$ ;

— $R_3$ and $R_4$, which are identical or different, denote a hydrogen atom or an atom or a group such as those denoted by $R_2$ ; in a molten medium, using gaseous chlorine, characterized in that it is carried out in the presence of an effective amount of at least one organic cation.

2. Process according to Claim 1, characterized in that it is carried out in the presence of at least one organic cation chosen from amongst the onium ions corresponding to one of the following general formulae :

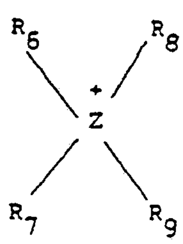

(II)

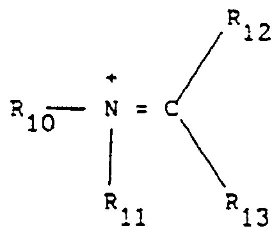

(III)

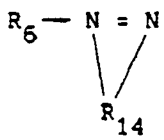

(III i )

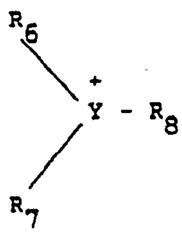

(IV)

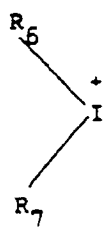

(V)

in which :

— Z denotes N, P or As ;

— Y denotes S, O, Se, S=O or C ;

— $R_6$, $R_7$, $R_8$ and $R_9$, which are identical or different, denote :

a straight-chain or branched alkyl radical having 1 to 16 carbon atoms and optionally substituted by one or more phenyl groups, hydroxyl groups, halogen atoms, nitro groups, alkoxy groups or alkoxycarbonyl groups, the alkoxy groups having 1 to 4 carbon atoms ;

a straight-chain or branched alkenyl radical having 2 to 12 carbon atoms ;

an aryl radical having 6 to 10 carbon atoms and optionally substituted by one or more alkyl groups having 1 to 4 carbon atoms, alkoxy groups, alkoxycarbonyl groups, the alkoxy radical having 1 to 4 carbon atoms, or halogen atoms,

two of the said radicals $R_6$ to $R_9$ being able to form together a straight-chain or branched alkylene, alkenylene or alkadienylene radical having 3 to 6 carbon atoms ;

— $R_{10}$, $R_{11}$, $R_{12}$, $R_{13}$ are identical or different and denote :

a straight-chain or branched alkyl radical containing 1 to 4 carbon atoms ;

the radicals $R_{12}$ and $R_{13}$ being able to form together an alkylene radical containing 3 to 6 carbon atoms;

the radicals $R_{11}$ and $R_{12}$ or $R_{11}$ and $R_{13}$ being able to form together an alkylene, alkenylene or alkadienylene radical containing 4 carbon atoms and forming with the nitrogen atom a nitrogen-containing heterocycle ;

— $R_{14}$ denotes a divalent radical forming with the two nitrogen atoms a ring having 4 to 6 atoms and being able to contain one or more nitrogen, sulphur and/or oxygen atoms, it being possible for the said ring to be substituted by one or more radicals such as $R_6$.

3. Process according to either of Claims 1 and 2, characterized in that the anion which is bound to the

organic cations is chosen from amongst the ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, Ph-$SO_3^-$, $HSO_4^-$, $NO_3^-$, $SO_4^{2-}$, $Cl^-$, $Br^-$, $I^-$, $OH^-$, Ph denoting a phenyl radical.

4. Process according to either of Claims 1 and 2, characterized in that the anion which is bound to the organic cations is preferably chosen from amongst the ions : $PO_4^{3-}$ ; $HPO_4^{2-}$ ; $H_2PO_4^-$ ; $CH_3SO_3^-$ · Ph-$SO_3^-$ · $NO_3^-$ · $SO_4^{2-}$ ; $PF_6^-$ ; $Cl^-$ ; $Br^-$ ; $I^-$ ; Ph denoting a phenyl radical.

5. Process according to one of Claims 1 to 4, characterized in that the organic cation is preferably a quaternary ammonium, a quaternary phosphonium, a sulphonium or an iodonium.

6. Process according to one of Claims 1 to 5, characterized in that the organic cation is present in such an amount that the weight of onium salt relative to the weight of phenolic compound of formula (I) varies between 0.005% and 25%.

7. Process according to one of Claims 1 to 5, characterized in that the organic cation is present in such an amount that the weight of onium salt relative to the weight of the phenolic compound of formula (I) varies between 0.01 and 5%.

8. Process according to one of Claims 1 to 7, characterized in that it is applied to the phenolic compounds of general formula (I) in which :

— $R_2$ denotes :

a chlorine atom, a fluorine atom or a bromine atom ;
a methyl, ethyl, propyl, isopropyl or tertbutyl group ;
a methoxy or ethoxy group ;
an aldehyde group ;
an OH group ;
a CN group ;
an $NO_2$ group ;
an acetoxy group ;
an acetamido group ;
an ester group —$COOR_5$, $R_5$ denoting a methyl or ethyl group ;
an acyl group having 2 to 4 carbon atoms ;

— $R_1$ denotes a hydrogen atom or has the meanings indicated for $R_2$ ;

— $R_3$ and $R_4$ denote :

one a hydrogen atom ;
the other a hydrogen atom or a group such as those denoted by $R_2$.

9. Process according to one of Claims 1 to 8, characterized in that it is applied to the phenolic compounds chosen from amongst 4-chlorophenol, 2,4-dichlorophenol, 3,4-dichlorophenol, 2-chloro-4-fluorophenol, 4-chloro-2-fluorophenol, 2-bromo-4-chlorophenol, 4-bromo-2-chlorophenol, 2-chloro-4-methoxyphenol, 4-chloro-2-methoxyphenol, 4-chloro-2-methylphenol, 4-methoxyphenol, 4-ethoxyphenol, 4-hydroxyacetophenone, 4-hydroxybenzonitrile, 3-chloro- 4-hydroxybenzaldehyde, 5-chloro-2-hydroxybenzaldehyde, 2-chloro-4-methylphenol, 2-chloro-4-tert-butylphenol, 2-chloro-4-nitrophenol, 4-chloro-2-nitrophenol, 4-nitrophenol, 2,4-dinitrophenol, 2-acetamido-4-chlorophenol, 4-acetamido-2-chlorophenol, 4-tertbutylphenol, 4-methylphenol, 4-ethylphenol, 4-isopropylphenol.

10. Process according to one of Claims 1 to 9, characterized in that it is applied to a mixture of 2,4-dichlorophenol and 2,6-dichlorophenol.

11. Process according to one of Claims 1 to 10, characterized in that it is applied to an industrial mixture containing 2,4-dichlorophenol,2,6-dichlorophenol and 2,4,6-trichlorophenol.

12. Process according to one of Claims 1 to 11, characterized in that it is carried out at a temperature less than or equal to 150°C, and preferably between the melting point of the reaction mixture and 120°C.